# EUROPEAN PATENT SPECIFICATION

(11) **EP 2 478 012 B1**
(45) Date of publication and mention of the grant of the patent: **09.11.2016**
(21) Application number: 10751563.7
(22) Date of filing: 26.08.2010
(51) Int. Cl.: C07K 16/28, A61P 37/06

(54) **TREATMENT OF AUTOIMMUNE AND INFLAMMATORY DISEASES WITH EPRATUZUMAB**
BEHANDLUNG VON AUTOIMMUNKRANKHEITEN UND ENTZÜNDLICHEN ERKRANKUNGEN MIT EPRATUZUMAB
TRAITEMENT DES MALADIES AUTOIMMUNES ET INFLAMMATOIRES AVEC EPRATUZUMAB

(30) Priority: 18.09.2009 US 243797 P
(43) Date of publication of application: 25.07.2012
(73) Proprietor: UCB Biopharma SPRL, 1070 Brussels (BE)
(72) Inventor: NOVOTNEY-BARRY, Anna-Marie, Cartersville GA 30120 (US); HULHOVEN, Réginald, B-3020 Veltem-Beisem (BE); PARKER, Gerald, L., Berkshire SL6 9SY (GB); HOSKIN, Violet, A., Lilburn GA 30047 (US)
(74) Representative: UCB Intellectual Property
(86) International application number: PCT/EP2010/005225
(87) International publication number: WO 2011/032633

(56) References cited:
- DÖRNER THOMAS ET AL: "Initial clinical trial of epratuzumab (humanized anti-CD22 antibody) for immunotherapy of systemic lupus erythematosus." ARTHRITIS RESEARCH & THERAPY, vol. 8, no. 3, R74, 2006, pages 1-11, XP002609526 ISSN: 1478-6362 DOI: 10.1186/ar1942
- DÖRNER THOMAS ET AL: "Targeting CD22 as a strategy for treating systemic autoimmune diseases." THERAPEUTICS AND CLINICAL RISK MANAGEMENT, vol. 3, no. 5, October 2007 (2007-10), pages 953-959, XP002609527 ISSN: 1176-6336
- ANOLIK JENNIFER H ET AL: "New treatments for SLE: cell-depleting and anti-cytokine therapies." BEST PRACTICE & RESEARCH. CLINICAL RHEUMATOLOGY, vol. 19, no. 5, October 2005 (2005-10), pages 859-878, XP5140891 ISSN: 1521-6942
- JUWEID M: "TECHNOLOGY EVALUATION: EPRATUZUMAB, IMMUNOMEDICS/AMGEN" CURRENT OPINION IN MOLECULAR THERAPEUTICS, CURRENT DRUGS, LONDON, GB, vol. 5, no. 2, 1 April 2003 (2003-04-01), pages 192-198, XP008022448 ISSN: 1464-8431
- LOONEY R JOHN ET AL: "A perspective on B-cell-targeting therapy for SLE." MODERN RHEUMATOLOGY / THE JAPAN RHEUMATISM ASSOCIATION, vol. 20, no. 1, February 2010 (2010-02), pages 1-10, XP002609529 ISSN: 1439-7609
- SUMAPA CHAIAMNUAY ET AL.: "The impact of increased body mass index on systemic lupus erythematosus", JOURNAL OF CLINICAL RHEUMATOLOGY, vol. 13, no. 3, 1 June 2007 (2007-06-01), pages 128-133,

## Description

### FIELD OF THE INVENTION

The present invention relates to the treatment of autoimmune and inflammatory diseases, in particular systemic lupus erythematosus, with anti-CD22 antibodies, in particular epratuzumab.

### BACKGROUND OF THE INVENTION

Autoimmune diseases comprise more than 80 chronic diseases that affect about 5%-8% of the general population. There has been considerable progress made in understanding the immune system during recent decades, resulting in a better appreciation of the role of B-cells in the interaction of innate and adaptive immunity, lymphocyte activation and antigen processing, the principles of immune tolerance, B- and T-cell crosstalk, cytokine signaling, and new approaches of treating autoimmune diseases by depleting or modulating B-cells, including blockade of co-stimulation. B-cells are considered as being of central importance in the immunopathogenicity of autoimmune diseases such as rheumatoid arthritis, seronegative spondyloarthropathies, primary Sjögren's syndrome, vasculitis and systemic lupus erythematosus (SLE). B-cells represent a target for the treatment of autoimmune disorders. To date, there are a number of therapeutic antibodies targeting B-cell-specific antigens in order to deplete or modulate B-cells, rituximab (anti-CD20 chimeric antibody), ocrelizumab (humanized anti-CD20 antibody), ofatumumab (human anti-CD20 antibody) and belimumab (anti-BlyS or BAFF human antibody). Clinical studies with rituximab indicated that circulating B-cells are undetectable after a brief dosing regimen of rituximab. Depleting B-cells with anti-CD20 antibodies is a new method in the treatment of autoimmune diseases. The sustained immunosuppression associated with B-cell depletion poses, however, risks on the patient as regards the increased occurrence of infectious and neoplastic diseases. To date limited long-term safety data are available. There is therefore a need in the art to develop a treatment for autoimmune disorders having B-cell involvement, such as rheumatoid arthritis, SLE, Sjögren's syndrome or vasculitis, which involves the modulation of B-cell activity without profound B-cell depletion in order to increase the safety of the treatment.

Systemic lupus erythematosus (SLE) has been classified as an autoimmune disease that may involve many organ systems, as an inflammatory multisystem rheumatic disorder, or as a collagen vascular disease. In Europe and the United States of America, estimates of the number of affected individuals range from 24 to 65 cases per 100,000 population in some studies. Predisposing factors for lupus include Asian or African race, and female gender. 90% of patients with lupus are female and the onset of symptoms usually occurs between the ages of 15 and 50 years. Systemic lupus erythematosus appears not to be a homogeneous disease, but a group of related syndromes, with widely varying presentations, degrees of body system involvement, and clinical course. Clinical features commonly seen in SLE are blood and lymphatic disorders (lymphadenopathy), cardiac disorders (e.g. cardiomyopathy, pericardial effusion, pericarditis), eye disorders (e.g. keratoconjunctivitis sicca), gastrointestinal disorders (e.g. mouth ulceration, pancreatitis, peritonitis, pharyngitis), general disorders (e.g. malaise, fatigue, pyrexia, weight decrease), nervous system disorders (e.g. cerebrovascular accident, cognitive disorder, migraine, headache, peripheral neuropathy), musculoskeletal and connective tissue disorders (e.g. arthralgia, arthritis (not erosive or destructive), fibromyalgia, fracture, myositis, osteonecrosis, osteoporosis, osteopenia), psychiatric disorders (e.g. affective disorder, anxiety, depression, neurosis, mental disorder due to a general medical condition, psychotic disorder), renal and urinary disorders (e.g. lupus nephritis, nephrotic syndrome), respiratory, thoracic, and mediastinal disorders (e.g. pleurisy, pneumonitis, pulmonary hypertension), skin and subcutaneous tissue disorders (e.g. alopecia, cutaneous lupus erythematosus, dermatitis, generalised erythema, livedo reticularis, panniculitis, rash maculo-papular, systemic lupus erythematosus rash, urticaria) and vascular disorders (e.g. hypertension, Raynaud's phenomenon, telangiectasis, thrombocytopenia, thrombophlebitis, vasculitis). Additionally, most SLE patients present with abnormal antibody patterns, including the presence of anti-nuclear- (ANA) and anti-double stranded DNA (anti-dsDNA) antibodies.

The clinical course of SLE is episodic, with flares recurring upon increasing underlying disability and organ damage. Corticosteroids are the cornerstone of treatment but are associated with an extensive number of side effects most frequently seen during long-term use. Other drugs used in the setting of lower-level activity include analgesics, nonsteroidal anti-inflammatory drugs (NSAIDs), local steroids, and antimalarial drugs (e.g., chloroquine or hydroxychloroquine), with common supportive medications including vasodilators (calcium channel blockers, angiotensin-converting enzyme [ACE] inhibitors) for renal hypertension or Raynaud's syndrome, local treatments for rashes or sicca syndromes, transfusions, intravenous (i.v.) globulin for cytopenias, anticonvulsants, antimigraine medications, anticoagulants for recurrent thromboses, and antidepressants. High-dose corticosteroids, e.g., 0.5 to 1.0 mg/kg/day oral prednisone (or equivalent) or 500 mg to 1 g daily pulse i.v. methylprednisolone, are used to manage acute SLE flares, with immunosuppressants (e.g., azathioprine, cyclophosphamide, methotrexate, mycophenolate mofetil, leflunomide) generally used in moderate and severe cases when other treatments are ineffective or to limit or prevent long-term major organ damage from the disease or corticosteroid use ('steroid-sparing'). This present therapeutic armamentarium is inadequate because of limited efficacy and/or adverse events profile. Despite the high medical need for new effective therapies of SLE with an good safety profile the development of such therapies has proven to be particularly difficult and many therapeutic candidates have failed.(Eisenberg, 2009)

The sialoadhesin CD22 is a member of a group of cell adhesion molecules within the immunoglobulin superfamily that display binding to glycans with terminal sialic acid residues. CD22 is a 130-kDa protein containing seven extracellular immunoglobulin-like domains, a short transmembrane sequence, and a 78-amino acid cytoplasmic tail. CD22 is a cell-surface glycoprotein that is uniquely located on B-cells and B-cell-derived tumor cells. Upon activation of B-cells, the expression level of cell-surface CD22 initially increases, but is subsequently down-regulated upon differentiation into antibody-producing cells. The essential role of CD22 in B-cell activation offers an excellent possibility for the development of agents that interfere with B-cell-mediated immune responses.

The murine monoclonal antibody, LL2 (originally named EPB-2), is a B-cell (CD22)-specific IgG₂ₐ monoclonal antibody generated against Raji Burkitt lymphoma cells, and found to be highly selective for normal B-cells and B-cell tumors, but not reactive with Hodgkin's disease, solid tumors, or non-lymphoid tissues (Pawlak-Byczkowska et al., 1989). After the chimerization of LL2 (Leung et al., 1994) a humanized IgG₁(_{κ}) form of the murine LL2, was developed for clinical use and named epratuzumab (hLL2) (Leung et al., 1995). The construct encoding epratuzumab was created by grafting the complementarity-determining regions (CDR) of the marine parental origin antibody in a human Ig_{G1} genetic backbone. The resulting epratuzumab contains the original murine sequence only at the antigen-binding sites, comprising about 10% of the molecule, the remainder being the human framework sequences.

Limited open-label phase I clinical studies with epratuzumab have been performed in primary Sjögren's syndrome (Steinfeld et al., 2006) and SLE (Steinfeld and Youinou, 2006). In both studies a dosing regimen with 4 consecutive administrations once every other week of doses of 360 mg/m² body surface epratuzumab was applied. The results suggested a clinical effect of epratuzumab in the treatment of the above autoimmune disorders as determined by the several parameters including the British Isles Lupus Assessment Group (BILAG) score for SLE. Due to the limited number of patients involved and the fact that these pilot studies lacked a placebo control or active control arm, further studies are warranted to confirm efficacy of epratuzumab and in particular to determine a preferred dose and dosing regimen for the use of epratuzumab in the treatment of a autoimmune and inflammatory disease, in particular SLE. An open-label, single-center study of 14 patients with moderately active systemic lupus erythematosus with epratuzumab has been performed (Dörner et al., 2006). Patients received 360 mg/m² body surface epratuzumab intravenously every 2 weeks for 4 doses (also reviewed in Anolik et al., 2005 and Dörner and Goldenberg, 2007).

### SUMMARY OF THE INVENTION

A clinical phase IIb randomized, double-blind, placebo-controlled, dose and dose regimen-ranging study of the safety and efficacy of epratuzumab in serologically-positive SLE patients with active disease has been performed. This study is identified as UCB study SL0007.

Interestingly the trial indicated that when treating inflammation or autoimmune disease such as SLE with epratuzumab there is non-linear relationship between the dose administered and the efficacy observed, thus the highest level dose administered was not the most efficacious. In fact the relationship between dose and efficacy is better characterized as a bell shaped distribution. Surprisingly, at the highest doses given the efficacy was lower than that observed at intermediate doses and overall the response of patients at this high dose was only slightly above that observed for placebo. In addition looking at the BILAG Improvement scores at 8 and 12 weeks s the highest dose was poorer than placebo.

As a result of the study a new dosing regimen for epratuzumab in the treatment of autoimmune and inflammatory diseases, in particular SLE, has now been found. Surprisingly, the new dosing regimen given the best results does not require do administer the highest dose but an intermediate dose to a patient in need of treatment.

The invention pertains to epratuzumab for use in a treatment according to the claims.

Additionally, the study established the most effective dose for use in a treatment regime once every other week. Interestingly, even when administered once every other week (as 1200 mg per administration) this dose showed statically significant efficacy over the placebo.

### BRIEF DESCRIPTION OF THE DRAWINGS

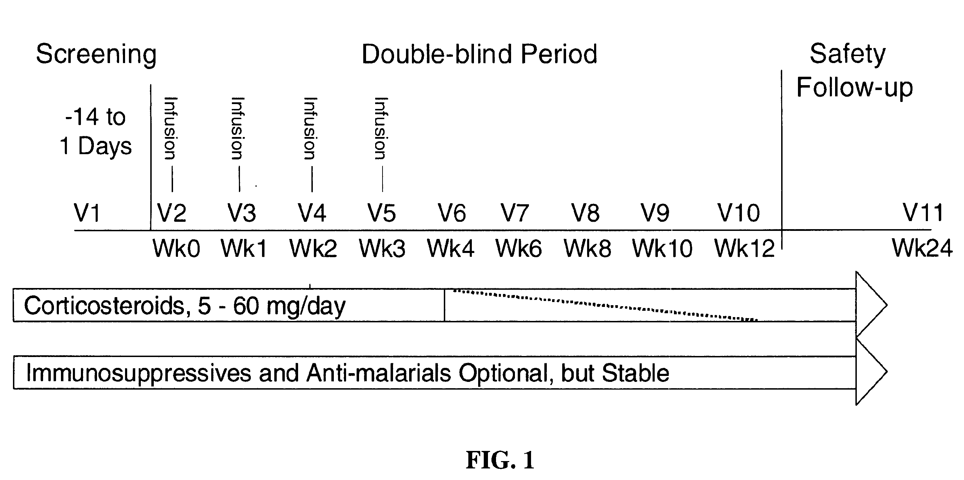

Figure 1 shows the study design. Patients with high moderate to severe disease activity as confirmed by the BILAG 2004 instrument were treated in a double-blind, placebo-controlled, randomized, adjunctive treatment design with dosing once every week for 4 blinded infusions total, 2 weeks or less screening period. Patients were required to have confirmed diagnosis of SLE by both American College of Rheumatology Criteria and laboratory markers of SLE (e.g. positive for anti-nuclear antibodies). Follow-up safety and efficacy assessments were done every 4 weeks. The primary endpoint (Combined Index Response) was measured at week 12. Corticosteroid taper was not required. The study was not powered for individual statistical comparisons among treatment arms. The aim of the study was to look for overall dose response by gathering data from many different dosing levels, and thus to enable identifying the best epratuzumab dose and dosing regimen.

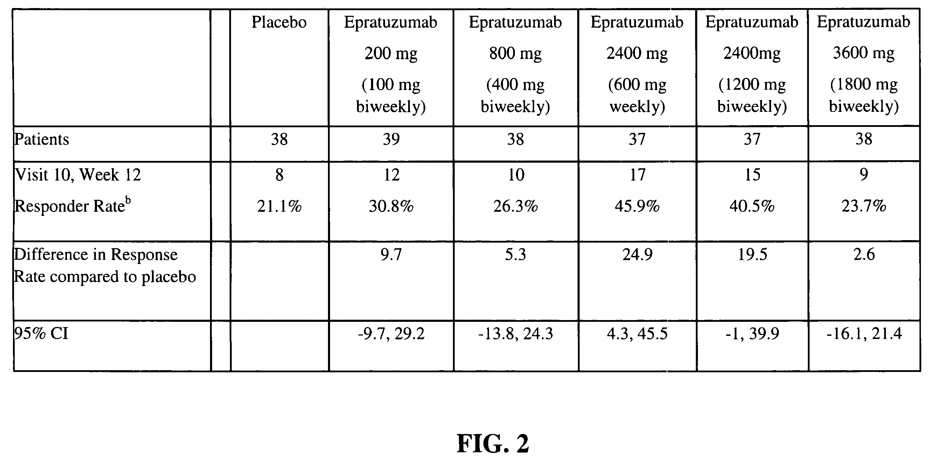

Figure 2 shows the Combined Index Response at Week 12 in the Intention to Treat (ITT) population based on Primary Efficacy Endpoint. Two of the 37 patients in the arm receiving 1200 mg epratuzumab once every other week (EOW) were randomized but never dosed. In the primary analysis, subjects who prematurely terminate the treatment period are classified as non-responders. The 95% confidence interval (CI) is provided.

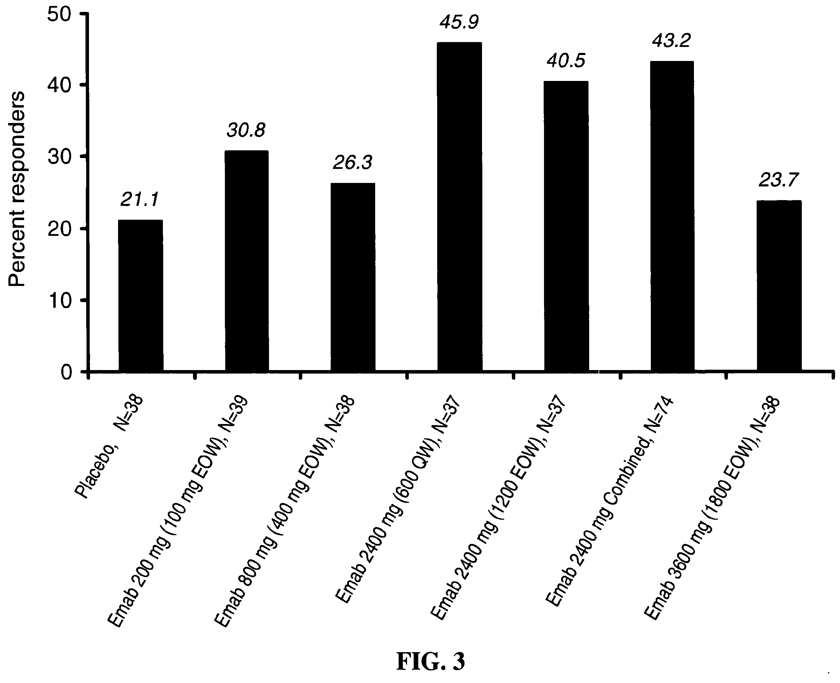

Figure 3 shows the percentage of responders in the ITT population as determined by the Combined Index Response Rate at Week 12 (primary efficacy variable). Two of the 37 patients in the arm receiving 1200 mg epratuzumab (Emab) once every other week (EOW) were randomized but never dosed. In the primary analysis, subjects who prematurely terminate the treatment period are classified as non-responders. The P-value for all 6 treatment arms for overall treatment effect assessed in primary analysis is P=0.148. P-values not adjusted for multiple comparisons.

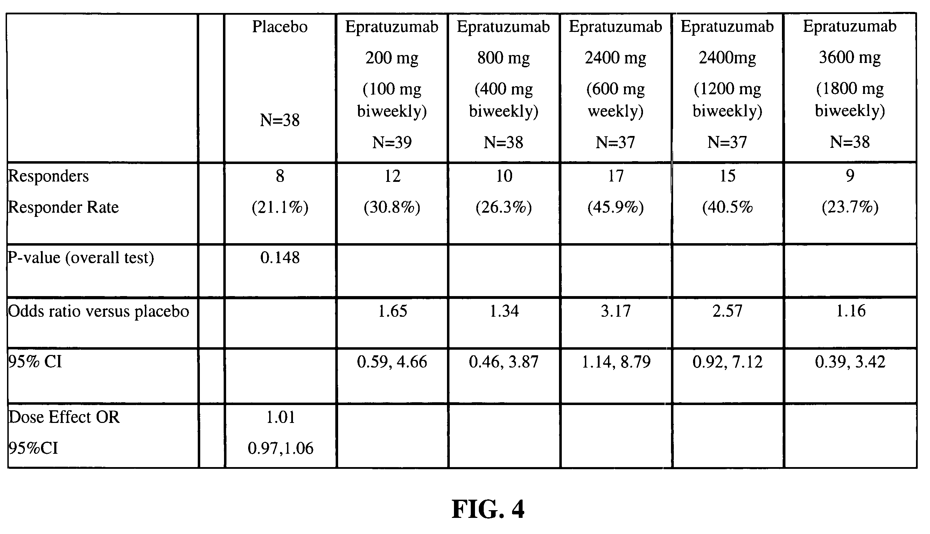

Figure 4 shows the Combined Index Response at Week 12 in the Intention to Treat (ITT) group analyzed by logistic regression-. Subjects who prematurely terminated the treatment period were classified as non-responders. A logistic model with factors for treat group, disease severity at baseline and concomitant immunosuppressive use at baseline was applied. Epratuzumab 600 mg once every week was analyzed as a dose of 1200 mg for the purpose of the odds ratio determination for dose effect. The 95% confidence interval (CI) is provided.

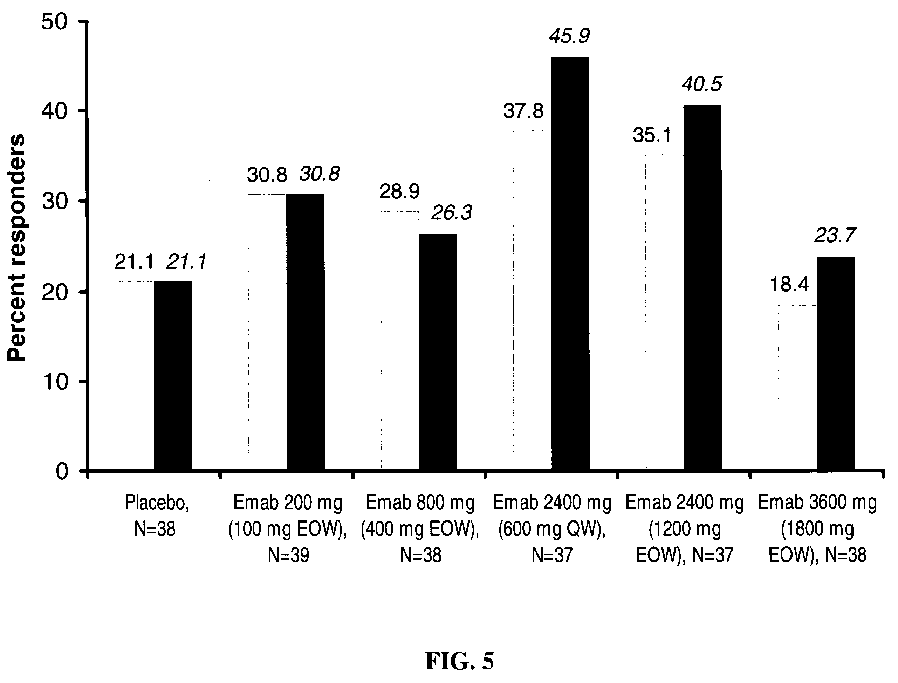

Figure 5 shows the percentage of responders as determined by the Combined Index Response Rates at Weeks 8 and 12. Already at week 8 response in the treatment arms with 600 mg Emab once every week (QW) and 1200 mg Emab once every other week (EOW) is better than in the other treatment arms.

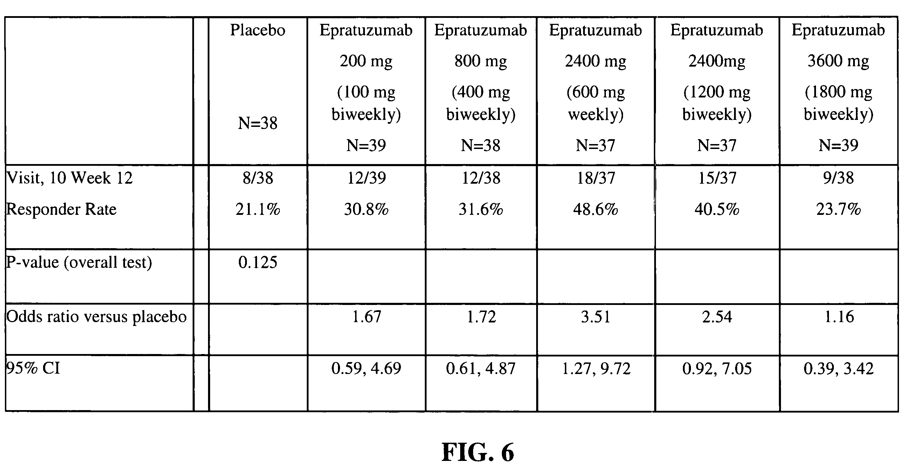

Figure 6 shows the Combined Index Response at Week 12 analyzed by logistic regression in the ITT group. Subjects with missing data at week 12 are imputed via last observation carried forward (LOCF). A logistic model with factors for treat group, disease severity at baseline and concomitant immunosuppressive use at baseline was applied. The 95% confidence interval (CI) is provided.

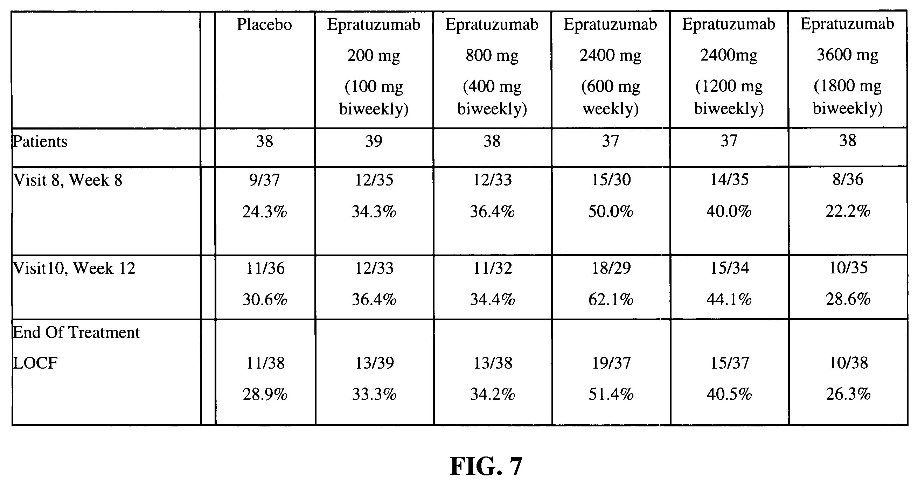

Figure 7 shows the BILAG Improvement by visit. BILAG Improvement defined as BILAG "A" scores at study entry improved to score "B"', "C"', "D" and BILAG "B" scores at study entry improved to score "C" or "D". Additionally, patients had to have 'no BILAG worsening' in other BILAG organ systems such that there are no new BILAG "A" score or two new BILAG "B" score.

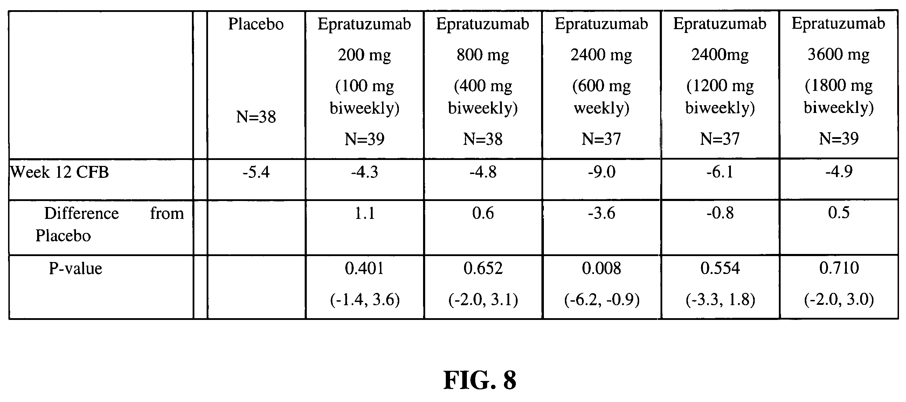

Figure 8 shows the BILAG Total Score- Least Square Mean Change from Baseline-Week 12. Further shown is the ANCOVA model with effects for treat group, baseline Total BILAG Score, and disease severity at baseline and concomitant immunosuppressive use at baseline.

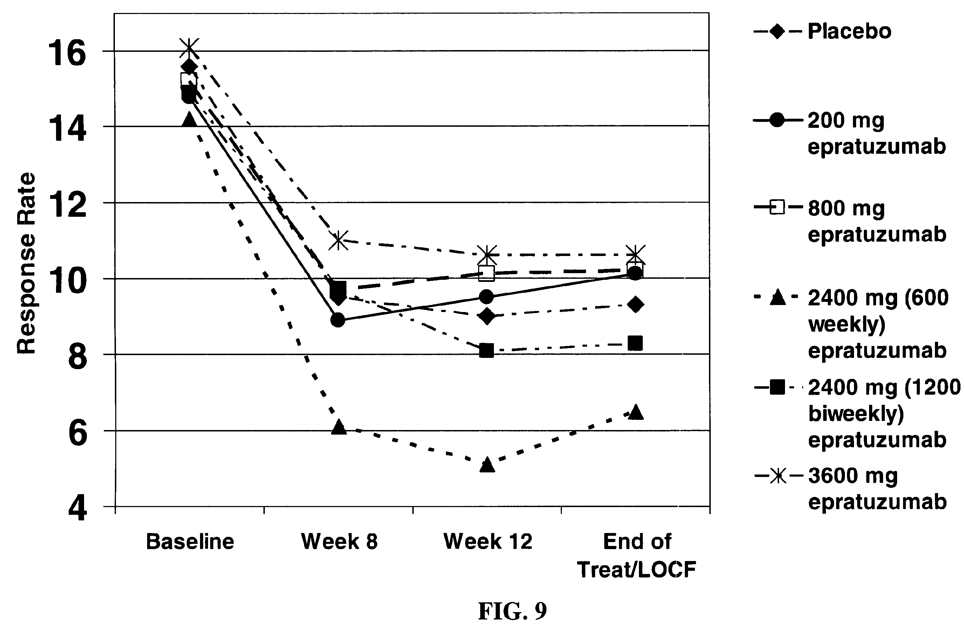

Figure 9 shows BILAG Total Score- Mean Score Over Time in the ITT group.

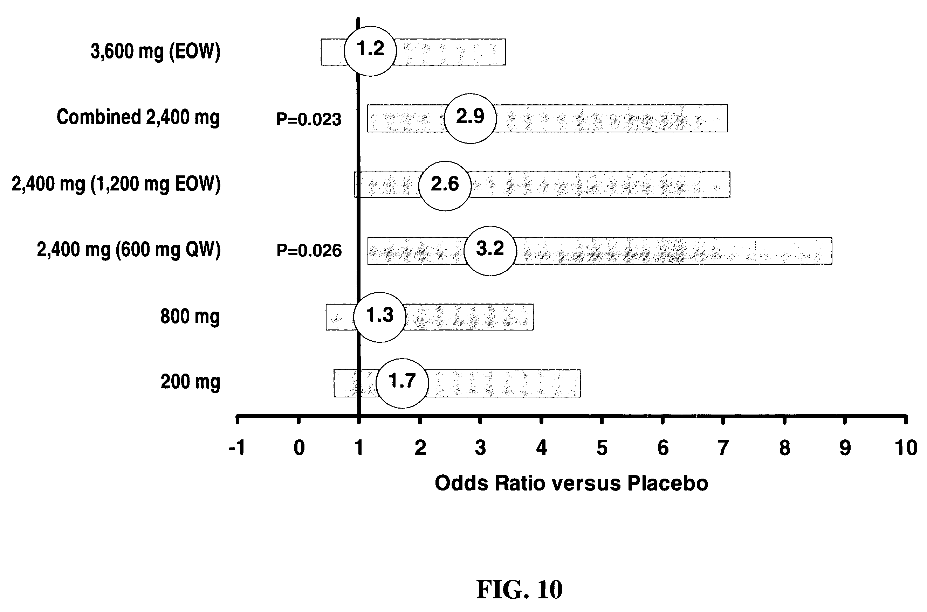

Figure 10 shows the odds ratio between the 6 treatment arms and placebo and the 95% confidence interval (CI). The odds ratio as well as the and 95% CI is significant higher for the treatment arms with 600 mg Emab once every week (QW) and 1200 mg Emab once every other week (EOW) as compared to the other treatment arms.

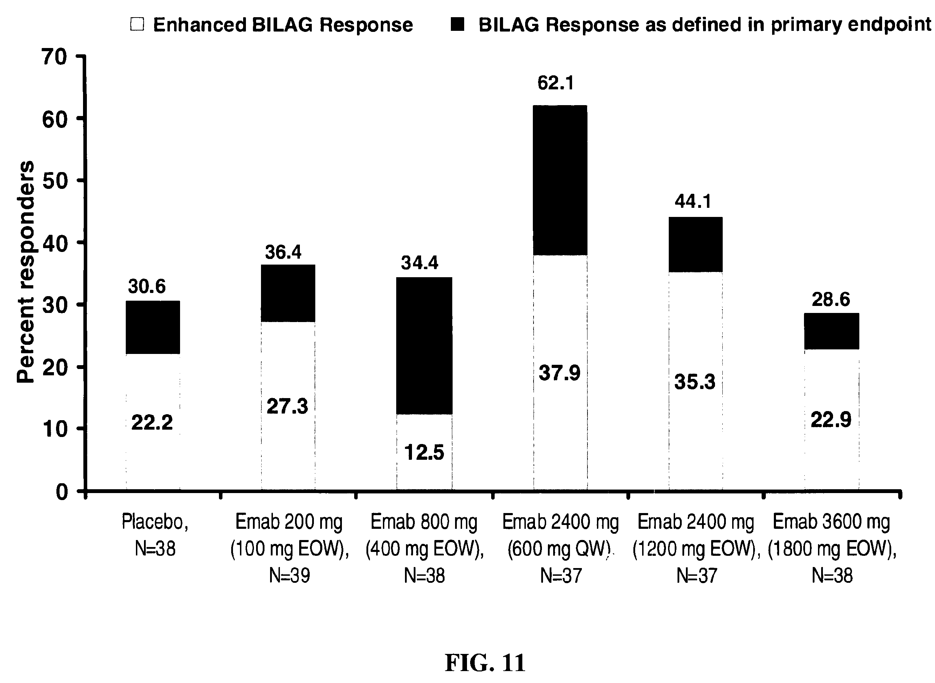

Figure 11 shows the BILAG and Enhanced BILAG Improvement at Week 12 in the treatment arms. BILAG Improvement is defined as BILAG A's at study entry improved to B/C/D and BILAG B's at study entry improved to C/D. Additionally, patients had to have 'no BILAG worsening' in other BILAG organ systems such that there are no new BILAG A's or two new BILAG B's. Enhanced BILAG response is defined as BILAG A's at study entry improved to C/D and BILAG B's at study entry improved to C/D. Additionally, for the Enhanced BILAG response patients had to have 'no BILAG worsening' in other BILAG organ systems such that there are no new BILAG A's or two new BILAG B's. The treatment arms with 600 mg Emab once every week (QW) and 1200 mg Emab once every other week (EOW) showed a remarkable BILAG Response as well as an Enhanced BILAG Response at Week 12.

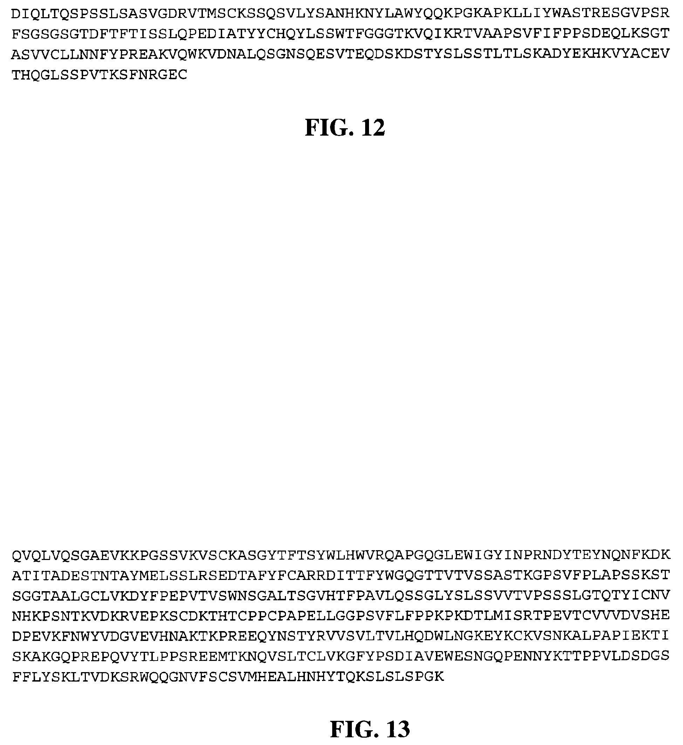

Figure 12 shows the amino acid sequence of the light chains of epratuzumab (SEQ ID NO:1).
Figure 13 shows the amino acid sequence of the heavy chains of epratuzumab (SEQ ID NO:2).

### DETAILED DESCRIPTION OF THE INVENTION

This invention pertains to methods of treating autoimmune or inflammatory diseases in which the administration of epratuzumab is beneficial. Embodiments of the invention relate to treatment of autoimmune or inflammatory diseases with epratuzumab.

A clinical phase IIb randomized, double-blind, placebo-controlled, dose and dose regimen-ranging study of the safety and efficacy of epratuzumab in serologically-positive SLE patients with active disease has been performed.

All epratuzumab treatment arms have superior response rates compared to placebo on primary endpoint measured after a treatment cycle at week 12. Surprisingly, the best response to treatment with epratuzumab was achieved with a particular dose and dosing regimen. The most effective dose and dosing regimen did not require the highest tested cumulative dose of 3600 mg but only 2400 mg cumulative dose. Both, the 4 times 600 mg once every week and the 2 times 1200 mg once every other week dosing regimen showed significantly higher efficacy as compared to the other study arms. Nevertheless, cumulative doses as low 200 mg showed efficacy in the trial.

The best effect in terms of response in patients with active SLE was achieved with the 4 times 600 mg epratuzumab once every week dosing in a 12 weeks treatment cycle.

In order that the present invention may be more readily understood, certain terms are first defined.

The term "dosing", as used herein, refers to the administration of a substance (e.g., epratuzumab), or a pharmaceutical composition comprising same, to achieve a therapeutic objective (e.g., the treatment of an autoimmune or inflammatory disease).

The term "cumulative dose", as used herein, refers to the total amount of epratuzumab administered over a defined period such as during one treatment cycle of 12 weeks.

The term "once every week", as used herein, in connection with treatment with, administration of or dosing of epratuzumab, or a composition comprising same, refers to the administration of epratuzumab or said composition every 5, 6 or preferably 7 days.

The term "once every other week", as used herein, in connection with treatment with, administration of or dosing of epratuzumab, or a composition comprising same, refers to the administration of epratuzumab or said composition every 9-19 days, more preferably, every 11-17 days, even more preferably, every 13-15 days, and most preferably, every 14 days. The dosing regimen with administration once every other week is not intended to include dosing regimen with administration once every week.

The term "treatment cycle", as used herein, refers to the period wherein epratuzumab is administered followed by a period with no administration of epratuzumab. Typically in a treatment cycle of 12 weeks epratuzumab is administered to a human subject in need of treatment within the first 4 weeks of the 12 weeks treatment cycle [e.g. once every week for 4 times (i.e. 4 administrations with an administration once once every week within the first 4 weeks) or once every other week (i.e. 2 administrations with an administration once every other week within the first 4 weeks] followed by the last 8 weeks of the treatment cycle with no administration of epratuzumab.

The term "epratuzumab", as used herein, refers to the humanized antibody known in the art under the International Non-Proprietary Name (INN) epratuzumab and described in US Patent No. 5,789,554 as humanized LL2. Epratuzmab has light chains having the amino acid sequence shown in SEQ ID NO:1 and heavy chains having the amino acid sequence shown in SEQ ID NO:2.

The term "BILAG score" or "BILAG" index refers to the British Isles Lupus Assessment Group score and index, respectively. The BILAG index was used to assess efficacy of treatment in patients with SLE in study SL0007. It is a comprehensive index for measuring SLE disease activity. The 2004 version of the BILAG index was used for the studies (Eisenberg, 2009; Isenberg et al., 2005). This version consists of 86 questions in 8 body systems (general, mucocutaneous, neurological, musculoskeletal, cardiovascular and respiratory, vasculitis, renal, and hematological). Some of the questions were based on the patient's history, some on examination findings, and others on laboratory results. Each body system score ranges from E to A, with A being the most severe disease activity. The interpretation of body system scores are as follows: A ("Active") = severely active disease (sufficient to require disease-modifying treatment, for example, greater than 20 mg/day of prednisone, immunosuppressants, cytoxics); B ("Beware") = moderately active disease (requires only symptomatic therapy, for example, less than or equal to 20 mg/day of prednisone or antimalarial drugs; C ("Contentment") = mild stable disease (no indication for changes in treatment); D = previously active disease - but none currently; E = no prior disease activity. When the BILAG alphabetic organ body system scores are converted to numeric values and summed (using the rule where each BILAG A=9, each BILAG B=3, each BILAG C=1, and each BILAG D or E is worth 0), this is referred to as a Total BILAG score.

The term "SLEDAI score" or "SLEDAI" index refers to the Systemic Lupus Erythematosus Disease Activity score/index, respectively (Hawker et al., 1993).

The term "autoimmune disease(s)" or "inflammatory disease(s)", as used herein, refers to autoimmune diseases or inflammatory diseases in which B-cells are implicated in the pathophysiology and/or the symptoms of disease. Such autoimmune diseases and inflammatory disease may also be referred to as B-cell mediated autoimmune diseases or inflammatory disease: B-cells have been implicated in playing a role in the pathophysiology of a variety of autoimmune or inflammatory diseases (Browning, 2006; Browning, 2006). For example, autoimmune diseases and inflammatory disease include but are not limited to rheumatoid arthritis, systemic lupus erythematosus, Sjögren's syndrome, ANCA-associated vasculitis, antiphospholipid syndrome, idiopathic thrombocytopaenia, autoimmune haemolytic anaemia, Guillian-Barré syndrome, chronic immune polyneuropathy, autoimmune thryoiditis, type I diabetes, Addison's disease, membranous glomerulonephropathy, Goodpasture's disease, autoimmune gastritis, pernicious anaemia, pemiphigus vulgarus, primary biliary cirrhosis, dermatomyositis-polymyositis, myasthenia gravis, celiac disease, immunoglobulin A nephropathy, Henoch-Schönlein purpura, chronic graft rejection, atopic dermatitis, asthma, allergy, systemic sclerosis, multiple sclerosis, Lyme neurobomeliosis, ulcerative colitis, interstitial lung disease.

In the first embodiment, the invention pertains to epratuzumab for use in treating systemic lupus erythematosus (SLE) wherein epratuzumab is administered intravenously in a particular fixed dose of 600 mg once every week for 4 times in a treatment cycle of 12 weeks, and wherein epratuzumab is administered in more than one treatment cycle of 12 weeks.

In another embodiment, the invention pertains to epratuzumab for use in treating systemic lupus erythematosus (SLE) according to the first embodiment, wherein epratuzumab is for administration at a concentration 10 mg/ml epratuzumab.

Further disclosed herein is epratuzumab, or a composition comprising same, for use in treating an autoimmune or inflammatory disease wherein epratuzumab is for administration in an amount of 400 to 800 mg once every week for 4 times in treatment cycle of 4 to 20 weeks, preferably 8 to 16 weeks, for example 9, 10, 11, 12, 13, 14 or 15 weeks, and more preferably 12 weeks.

Further disclosed herein is epratuzumab, or a composition comprising same, for use in treating an autoimmune or inflammatory disease as disclosed herein, wherein epratuzumab, or a composition comprising same, is for administration in an amount of 500 to 700 mg.

Further disclosed herein is epratuzumab, or a composition comprising same, for use in treating an autoimmune or inflammatory disease as disclosed herein, wherein epratuzumab, or a composition comprising same, is for administration in an amount of 550 to 650 mg.

Further disclosed herein is epratuzumab, or a composition comprising same, for use in treating an autoimmune or inflammatory disease wherein epratuzumab, or a composition comprising same, is for administration in an amount of 1000 to 1400 mg once every other week for 2 times in a treatment cycle of 4 to 20 weeks, preferably 8 to 16 weeks, for example 9, 10, 11, 12, 13, 14 or 15 weeks, and more preferably 12 weeks.

Further disclosed herein is epratuzumab, or a composition comprising same, for use in treating an autoimmune or inflammatory disease as disclosed herein, wherein epratuzumab, or a composition comprising same, is for administration in an amount of 1100 to 1300 mg.

Further disclosed herein is epratuzumab, or a composition comprising same, for use in treating an autoimmune or inflammatory disease as disclosed herein, wherein epratuzumab, or a composition comprising same, is for administration in an amount of 1150 to 1250 mg.

Further disclosed herein is epratuzumab, or a composition comprising same, for use in treating an autoimmune or inflammatory disease as disclosed herein, wherein epratuzumab, or a composition comprising same, is for administration in an amount of 1200 mg.

Treatment of chronic disorders such as inflammatory and autoimmune diseases, including SLE, may involve repetition of treatment cycles. Treatment cycles may be repeated e.g. over several years; e.g. 12 week treatment cycles as disclosed herein may be repeated such that treatment of an inflammatory and autoimmune diseases

Further disclosed herein is epratuzumab, or a composition comprising same, for use in treating an autoimmune or inflammatory disease as disclosed herein, wherein epratuzumab, or a composition comprising same, is for administration in more than one treatment cycle of 4 to 20 weeks, preferably 8 to 16 weeks, and more preferably 12 weeks.

Further disclosed herein is epratuzumab, or a composition comprising same, for use in treating an autoimmune or inflammatory disease as disclosed herein, wherein epratuzumab, or a composition comprising same, is for intravenous administration.

Further disclosed herein is epratuzumab, or a composition comprising same, for use in treating an autoimmune or inflammatory disease as disclosed herein, wherein epratuzumab, or a composition comprising same, is for administration at a concentration of 8 to 12 mg/ml, preferably 9 to 11 mg/ml and preferably 10 mg/ml epratuzumab.

Further disclosed herein is epratuzumab, or a composition comprising same, for use in treating an autoimmune or inflammatory disease as disclosed herein, wherein epratuzumab, or a composition comprising same, is for subcutaneous administration.

Further disclosed herein is epratuzumab, or a composition comprising same, for use in treating an autoimmune or inflammatory disease as disclosed herein, wherein epratuzumab, or a composition comprising same, is for intramuscular administration.

Further disclosed herein is epratuzumab, or a composition comprising same, for use in treating an autoimmune or inflammatory disease as disclosed herein, wherein the disease is rheumatoid arthritis, systemic lupus erythematosus, Sjögren's syndrome or vasculitis.

Further disclosed herein is epratuzumab, or a composition comprising same, for use in treating an autoimmune or inflammatory disease as disclosed herein, wherein epratuzumab, or a composition comprising same, is for administration in at least 2 treatment cycles of 10 to 14 weeks, preferably of 12 weeks. Further disclosed herein is epratuzumab, or a composition comprising same, for administration in 3, 4, 5, 6, 7, 8, 10, 12, 15, 20, 30, 40, 50 or more treatment cycles, including a life-long repetition of treatment cycles as disclosed herein.

Further disclosed herein is invention pertains to epratuzumab, or a composition comprising same, for use in treating an autoimmune or inflammatory disease comprising administering epratuzumab, or a composition comprising same, wherein epratuzumab, or a composition comprising same, is for administration once every week or once every other week at a cumulative dose of 2400 mg epratuzumab in a treatment cycle of 4 to 20 weeks, preferably 8 to 16 weeks, for example 9, 10, 11, 12, 13, 14 or 15 weeks, and more preferably 12 weeks.

Further disclosed herein is epratuzumab, or a composition comprising same, for use in treating an autoimmune or inflammatory disease as disclosed herein, wherein epratuzumab, or a composition comprising same, is for administration once every week at a dose of 600 mg epratuzumab or once every other week at a dose of 1200 mg epratuzumab.

Further disclosed herein is epratuzumab, or a composition comprising same, for use in treating an autoimmune or inflammatory disease as disclosed herein, wherein the autoimmune or inflammatory disease is rheumatoid arthritis, SLE, Sjögren's syndrome or vasculitis.

Further disclosed herein is epratuzumab, or a composition comprising same, for use in treating an autoimmune or inflammatory disease as disclosed herein, wherein epratuzumab, or a composition comprising same, is for administration in more than one treatment cycle of 4 to 20 weeks, preferably 8 to 16 weeks, for example 9, 10, 11, 12, 13, 14 or 15 weeks, and more preferably 12 weeks.

Further disclosed herein is a kit comprising epratuzumab, or a composition comprising same, in an amount as described herein, and instructions for dosing of epratuzumab as disclosed herein, respectively.

Further disclosed herein is epratuzumab, or a composition comprising same, for use in treating an autoimmune or inflammatory disease, in particular rheumatoid arthritis, SLE, Sjögren's syndrome, vasculitis in combination with other active compounds.

Further disclosed herein is the incorporation of additional active compounds into the composition comprising epratuzumab according to the invention. Further disclosed herein is the coformulation and/or coadministration of epratuzumab with one or more additional therapeutic agents. For example, epratuzumab may be coformulated and/or coadministered with a corticosteroid, a non-steroidal anti-inflammatory drug (NSAIDs), chloroquine, hydroxycloroquine, methotrexate, leflunomide, azathioprine, mycophenolate mofetil, cyclophosphamide, chlorambucil, and cyclospoune, mycophenolate mofetil, a CD20 antagonist, such as rituximab, ocrelizumab, veltuzumab or ofatumumab, abatacept, a TNF antagonist, such as etanercept, tacrolimus, dehydroepiandrosterone, lenalidomide, a CD40 antagonist, such as anti-CD40L antibodies, abetimus sodium and/or belimumab.

Further disclosed herein is epratuzumab, or a composition comprising same, for use as disclosed herein in combination with one or more of the foregoing therapeutic agents. Such combination therapies may advantageously utilize lower dosages of the administered therapeutic agents, thus avoiding possible toxicities or complications associated with the various monotherapies.

Epratuzumab for use as disclosed herein can be derivatized or linked to another functional molecule (e.g., a toxin or radionuclide). Accordingly, the antibodies and antibody portions of the invention are intended to include derivatized and otherwise modified forms of epratuzumab described herein. For example, epratuzumab can be functionally linked (by chemical coupling, genetic fusion, noncovalent association or otherwise) to one or more other molecular entities, such as a detectable agent, a cytotoxic agent, a pharmaceutical agent, and/or a protein or peptide that can mediate association of epratuzumab with another molecule (such as a streptavidin core region or a polyhistidine tag).

Epratuzumab for use as disclosed herein is optionally conjugated to another agent, such as a cytotoxic agent (e.g. a toxin such as diphtheria toxin, maytansine, maytansinoid, doxorubicin, calicheamicin, ozogamicin, auristatin, a derivative of auristatin (e.g. monomethyl auristatin), Pseudomonas exotoxin, ricin, ricin A chain, brin, abrin, mistletoe lectin, modeccin, pokeweed antiviral protein, PAP, saporin, bryodin 1, bouganin, gelonin, or alpha-sarcin), a radionuclide (e.g. scandium-47, copper-64, copper-67, gallium-67, yttrium-90, yttrium-91, palladium-103, rhodium-105, indium-111, tin-117, iodine-125, iodine-131, samarium-153, dysprosium-166, holmium-166, ytterbium-175, rhenium-186, rhenium-188, lutetium-177, indium-192, osmium-194, gold-198 or bismuth- 213) or a cytokine (for example IL-2 or TNF). Epratuzumab for use as disclosed herein may also be conjugated to a therapeutic agent such as a chemotherapeutic agent, therapeutic polypeptide, nanoparticle, liposome or therapeutic nucleic acid, or to imaging agent such as an enzyme, radionuclide or fluorophore.

Epratuzumab can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in US Patent No. 4,816,397.

To express epratuzumab, DNAs encoding the light and heavy chains, obtained by recombinant DNA techniques known in the art, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the epratuzumab genes. The expression vector and expression control sequences are chosen to be compatible with the expression host cell used. The epratuzumab light chain gene and the epratuzumab heavy chain gene can be inserted into separate vector or, more typically, both genes are inserted into the same expression vector. The antibody genes are inserted into the expression vector by standard methods (e.g., ligation of complementary restriction sites on the epratuzumab gene fragment and vector, or blunt end ligation if no restriction sites are present). The recombinant expression vector can encode a signal peptide that facilitates secretion of the antibody chain from a host cell. The antibody chain gene can be cloned into the vector such that the signal peptide is linked in-frame to the amino terminus of the epratuzumab chain gene. The signal peptide can be an immunoglobulin signal peptide or a heterologous signal peptide (i.e., a signal peptide from a non-immunoglobulin protein).

In addition to the antibody chain genes of epratuzumab, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel; Gene Expression Technology: Methods in Enzymology 185, Academic Press, San Diego, Calif. (1990). It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, [e.g., the adenovirus major late promoter (AdMLP)] and polyoma.

In addition to the antibody chain genes and regulatory sequences, the recombinant expression vectors may carry additional sequences, such as sequences that regulate replication of the vector in host cells (e.g., origins of replication) and selectable marker genes. The selectable marker gene facilitates selection of host cells into which the vector has been introduced (see e.g., US Patent No. 5,179,017).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody.

Preferred mammalian host cells for expressing the recombinant epratuzumab for use according to the invention include Chinese Hamster Ovary (CHO cells), NSO myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

In a preferred system for recombinant expression of epratuzumab, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Preferred compositions suitable for administration to a human subject for the uses disclosed herein comprise epratuzumab and a pharmaceutically acceptable carrier, excipient, or stabilizer. As used herein, "pharmaceutically acceptable carrier" includes any and all solvents, dispersion media, coatings, antibacterial and antifungal agents, isotonic and absorption delaying agents, and the like that are physiologically compatible and are suitable for administration to a subject for the uses described herein. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl-parabens such as methyl- or propyl-paraben; catechol; resorcinol; cyclohexanol; 3-pentanol; and m-cresol); low molecular weight (not more than about 10 amino acid residues) peptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN, PLURONICS or polyethylene glycol.

Therapeutic compositions typically must be sterile and stable under the conditions of manufacture and storage. The composition can be formulated in solution or freeze-dried form.

Epratuzumab can be used to treat autoimmune or inflammatory diseases with the dose and according to the dosing regimen disclosed herein, in particular rheumatoid arthritis, rheumatoid spondylitis, seronegative spondyloarthropathies, psoriasis, psoriatic arthritis, systemic lupus erythematosus, Sjögren's syndrome, vasculitis, allergy, multiple sclerosis, type I diabetes, autoimmune uveitis and nephrotic syndrome.

Comprising in the context of the present specification is intended to meaning including.

The invention will now be described with reference to the following examples, which are merely illustrative and should not in any way be construed as limiting the scope of the present invention.

### EXAMPLE 1

Treatment of SLE patients with active disease in phase IIb randomized, double-blind, placebo-controlled, dose and dose regimen-ranging study of the safety and efficacy of epratuzumab.

In this study, 189 patients with active SLE were treated with epratuzumab according to the following scheme. 38 patients were treated with placebo.

| Number of patients | |
|---|---|
| 38 | Placebo (PBS) i.v. at weeks 0, 1, 2 & 3 |
| 39 | epratuzumab cumulative dose 200 mg (100mg i.v. at weeks 0 & 2; placebo at weeks 1 & 3) |
| 38 | epratuzumab cumulative dose 800 mg (400mg i.v. at weeks 0 & 2, placebo at weeks 1 & 3) |
| 37 | epratuzumab cumulative dose 2400 mg (1200mg i.v. at weeks 0 & 2, placebo at weeks 1 & 3) |
| 37 | epratuzumab cumulative dose 2400 mg (600mg* i.v. at weeks 0, 1, 2, & 3) |
| 38 | epratuzumab cumulative dose 3600 mg (1800mg i.v. at weeks 0 & 2, placebo at weeks 1 & 3) |

Epratuzumab was produced in a mammalian cell line (SP2/0 myeloma cells) transfected with a vector containing the sequence of the humanized antibody. The antibody-producing cells were grown in suspension in a controlled bioreactor. The cells were harvested and the antibody was purified using a series of chromatography steps. The purification process includes multiple inactivation and removal steps to ensure freedom from viral, retroviral, and bacterial contamination. Epratuzumab was formulated in 0.04 M sodium phosphate-0.15 M sodium chloride, pH 7.4, buffer with 0.075 % polysorbate 80. Epratuzumab was used as a sterile, clear, colorless, preservative-free liquid formulation in vials at a protein concentration of 9 to 11 mg/mL in phosphate buffered saline (PBS) with 0.075% polysorbate 80 in single-use dose form.

Epratuzumab was administered intravenously (i.v.) as a slow infusion (≤1 hour). Epratuzumab should not be administered as a bolus. The infusion rate may be slowed, interrupted, or terminated, if adverse reactions are being observed during infusion, as considered appropriate by the treating physician.

Patients participating in this study had a mean disease activity score (Total BILAG score [where A=9] of 15.2 and a mean of SLEDAI score of 14.8. 70% of patients had severe active SLE disease and 30% had moderate active SLE disease at baseline.

The clinical endpoint of study SL0007 was response as determined by the Combined Response Index at Week 12 of the study. All of the following criteria needed to be met for responders according to the Combined Response Index:
- BILAG Improvement: All BILAG scores "A" (severe body systems) at study entry needed to be improved to score "B", "C", or "D" and all BILAG scores "B" (moderate body systems) at study entry needed to be improved to score "C" or "D".
- No BILAG Worsening: No BILAG score should worsen in other body systems such that there are no new BILAG "A" scores or two new BILAG "B" scores.
- No SLEDAI total score worsening was allowed compared to study entry.
- No worsening in physician's global disease activity assessment was allowed as defined by less than 10% worsening on 100 mm VAS compared to study entry.

Cannot be treatment failures include patients who add or increase dose of immunosuppressants or antimalarials, or increase corticosteroids above baseline treatment level or tapering level.

All epratuzumab treatment arms have superior response rates compared to placebo on primary endpoint measured at week 12. Clinically meaningful treatment difference versus placebo was achieved for both the epratuzumab 2400 mg cumulative dose arms (600 mg once every week and 1200 mg once every other week). 2400 mg delivered as 4 divided doses once every week appears generally superior to 2400 mg given as 2 divided doses once every other week. The two low dosage epratuzumab arms of 200 mg, 800 mg, and the highest dose 3600 mg did not demonstrate a clinically meaningful response rate difference from placebo epratuzumab was well-tolerated and no significant new safety signals were identified among the adverse event data. Epratuzumab treatment resulted in a similar incidence of adverse events, infusion reactions, serious adverse events and infections compared to placebo. Furthermore, a low incidence of human antibodies against epratuzumab was detected (3 out of 187 exposed patients exhibited a human anti-human antibody to epratuzumab during the treatment phase of the study).

### REFERENCES

Anolik JH, Aringer M, (2005). New treatments for SLE: cell-depleting and anti-cytokine therapies, Best Pract Res Clin Rheumatol. Oct:19(5):859-78.
Browning,J.L. (2006). B cells move to centre stage: novel opportunities for autoimmune disease treatment. Nat Rev Drug Discov 5, 564-576.
Dörner T, Goldenberg DM (2007). Targeting CD22 as a strategy for treating systemic autoimmune diseases, Ther Clin Risk Manag. Oct;3(5):953-9
Dörner T, Kaufmann J, Wegener WA, Teoh N, Goldenberg DM, Burmester GR. (2006) Initial clinical trial of epratuzumab (humanized anti-CD22 antibody) for immunotherapy or systemic lupus erythematosus, Arthritis Res Ther.8(3):R74
Eisenberg,R. (2009). Why can't we find a new treatment for SLE? J Autoimmun. 32, 223-230.
Hawker,G., Gabriel,S., Bombardier,C., Goldsmith,C., Caron,D., and Gladman,D. (1993). A reliability study of SLEDAI: a disease activity index for systemic lupus erythematosus. J Rheumatol 20, 657-660.
Isenberg,D.A., Rahman,A., Allen,E., Farewell,V., Akil,M., Bruce,I.N., D'Cruz,D., Griffiths,B., Khamashta,M., Maddison,P., McHugh,N., Snaith,M., Teh,L.S., Yee,C.S., Zoma,A., and Gordon,C. (2005). BILAG 2004. Development and initial validation of an updated version of the British Isles Lupus Assessment Group's disease activity index for patients with systemic lupus erythematosus. Rheumatology (Oxford) 44, 902-906.
Leung,S.O., Goldenberg,D.M., Dion,A.S., Pellegrini,M.C., Shevitz,J., Shih,L.B., and Hansen,H.J. (1995). Construction and characterization of a humanized, internalizing, B-cell (CD22)-specific, leukemia/lymphoma antibody, LL2. Mol Immunol 32, 1413-1427.
Leung,S.O., Shevitz,J., Pellegrini,M.C., Dion,A.S., Shih,L.B., Goldenberg,D.M., and Hansen,H.J. (1994). Chimerization of LL2, a rapidly internalizing antibody specific for B cell lymphoma. Hybridoma 13, 469-476.
Pawlak-Byczkowska,E.J., Hansen,H.J., Dion,A.S., and Goldenberg,D.M. (1989). Two new monoclonal antibodies, EPB-1 and EPB-2, reactive with human lymphoma. Cancer Res 49, 4568-4577.
Steinfeld,S.D., Tant,L., Burmester.G.R., Teoh,N.K., Wegener,W.A., Goldenberg,D.M., and Pradier,O. (2006). Epratuzumab (humanised anti-CD22 antibody) in primary Sjogren's syndrome: an open-label phase I/II study. Arthritis Res Ther 8, R129.
Steinfeld,S.D. and Youinou,P. (2006). Epratuzumab (humanised anti-CD22 antibody) in autoimmune diseases. Expert Opin Biol Ther 6, 943-949.

## Claims

1. Epratuzumab for use in treating systemic lupus erythematosus (SLE) wherein epratuzumab is administered intravenously in a particular fixed dose of 600 mg once every week for 4 times at a cumulative dose of 2400 mg epratuzumab in a treatment cycle of 12 weeks, and wherein epratuzumab is administered in more than one treatment cycle of 12 weeks.

2. Epratuzumab according to claim 1, wherein epratuzumab is for administration in a composition at a concentration of 10 mg/ml.

## Patentansprüche

1. Epratuzumab zur Verwendung bei der Behandlung von systemischem Lupus erythematodes (SLE), wobei Epratuzumab intravenös in einer bestimmten festgelegten Dosis von 600 mg einmal jede Woche 4-mal in einer kumulativen Dosis von 2400 mg Epratuzumab in einem Behandlungszyklus von 12 Wochen verabreicht wird und wobei Epratuzumab in mehr als einem Behandlungszyklus von 12 Wochen verabreicht wird.

2. Epratuzumab nach Anspruch 1, wobei das Epratuzumab zur Verabreichung in einer Zusammensetzung in einer Konzentration von 10 mg/ml vorliegt.

## Revendications

1. Épratuzumab destiné à être utilisé dans le traitement du lupus érythémateux disséminé (SLE), l'épratuzumab étant administré par voie intraveineuse à une dose fixée particulière de 600 mg une fois toutes les semaines à quatre reprises à une dose cumulative de 2400 mg d'épratuzumab dans un cycle de traitement de 12 semaines, et l'épratuzumab étant administré dans plus d'un cycle de traitement de 12 semaines.

2. Épratuzumab selon la revendication 1, l'épratuzumab étant destiné à être administré dans une composition à une concentration de 10 mg/ml.


### SEQUENCE LISTING

<110> UCB Pharma SA
<120> TREATMENT OF AUTOIMMUNE AND INFLAMMATORY DISEASES
<130> B0069 WO
<150> US 61/243,797
   <151> 2009-09-18
<160> 2
<170> Patent In version 3.5
<210> 1
   <211> 219
   <212> PRT
   <213> Artificial Sequence
<220>
   <223> Epratuzumab light chain
<400> 1
   Asp Ile Gln Leu Thr Gln Ser Pro Ser Ser Leu Ser Ala Ser Val Gly
   1               5                   10                  15
   Asp Arg Val Thr Met Ser Cys Lys Ser Ser Gln Ser Val Leu Tyr Ser
               20                  25                  30
   Ala Asn His Lys Asn Tyr Leu Ala Trp Tyr Gln Gln Lys Pro Gly Lys
           35                  40                  45
   Ala Pro Lys Leu Leu Ile Tyr Trp Ala Ser Thr Arg Glu Ser Gly Val
       50                  55                  60
   Pro Ser Arg Phe Ser Gly Ser Gly Ser Gly Thr Asp Phe Thr Phe Thr
   65                  70                  75                  80
   Ile Ser Ser Leu Gln Pro Glu Asp Ile Ala Thr Tyr Tyr Cys His Gln
                   85                  90                  95
   Tyr Leu Ser Ser Trp Thr Phe Gly Gly Gly Thr Lys Val Gln Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 2
   <211> 446
   <212> PRT
   <213> Artificial Sequence
<220>
   <223> Epratuzumab heavy chain
<400> 2
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Thr Phe Thr Ser Tyr
   20 25 30
   Trp Leu His Trp Val Arg Gln Ala Pro Gly Gln Gly Leu Glu Trp Ile
   35 40 45
   Gly Tyr Ile Asn Pro Arg Asn Asp Tyr Thr Glu Tyr Asn Gln Asn Phe
   50 55 60
   Lys Asp Lys Ala Thr Ile Thr Ala Asp Glu Ser Thr Asn Thr Ala Tyr
   65 70 75 80
   Met Glu Leu Ser Ser Leu Arg Ser Glu Asp Thr Ala Phe Tyr Phe Cys
   85 90 95
   Ala Arg Arg Asp Ile Thr Thr Phe Tyr Trp Gly Gln Gly Thr Thr Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Arg Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445